# EUROPEAN PATENT APPLICATION

(11) **EP 0 711 574 A1**
(43) Date of publication of application: **15.05.1996**
(21) Application number: 95308016.5
(22) Date of filing: 09.11.1995
(51) Int. Cl.: A61M 25/00

(54) **Catheter with dual round lumens**

(30) Priority: 10.11.1994 US 339171
(71) Applicant: MED-PRO DESIGN, INC., Mississauga, Ontario L5A 3V3 (CA)
(72) Inventor: Martin, Geoffrey S, Mississauga, Ontario (CA)
(74) Representative: Bowles, Sharon Margaret

(57) **Abstract**

The invention provides catheters having round lumens arranged in side-by-side relationship and separated by a thin wall which enlarges into reinforcement zones to either end of the wall, the whole being contained within a generally elliptical outer surface. The catheter exhibits improved resistance to kinking.

## Description

### Field of the Invention

This invention relates to dual flow catheters of a type having an intake lumen to remove fluid and a return lumen to replace fluid.

The invention will be described with reference particularly to dual flow haemodialysis catheters but is not limited to such use. In haemodialysis, there are two types of dual lumen catheters used extensively. The first type is a coaxial catheter made up of inner and outer round tubes arranged to that the inner tube defines a central return lumen and the inner and outer tubes are spaced radially to define an annular intake lumen. Such a catheter has a structure at the proximal end for effecting connections between these lumens and external apparatus which causes flow into and from the catheter. Intake flow to the apparatus takes place through the annular lumen and return is through the central lumen. The arrangement is such that there is inevitably a large surface area in contact with the blood as it flows through the annular lumen. Also, the central lumen is free to find a natural position within the outer tube and this does not necessarily create a concentric intake lumen. In some circumstances the intake flow can be turbulent and subject to significant flow losses due to the large surface area involved. Nevertheless, such catheters have found acceptance for use in haemodialysis primarily because they permit the formation of intake openings around the complete circumference of the catheter and this tends to ensure that the catheter is never occluded by engagement with a blood vessel wall when in use.

The second type of catheter that has gained prominence is a side-by-side catheter in which the lumens are generally D-shaped and separated by a planar septum. The outer periphery is circular and blood flows through the D-shaped lumens. It is well established in the theory of liquid flow, that planar flow conditions are most readily achieved in a round cross-section. Since planar flow will reduce the potential for stress impact of the blood cells a round cross-section would be preferred. The double-D section has other disadvantages including the fact that the inlet openings must be to one side of the catheter and this means that all of the side openings for the intake lumen can be occluded if the catheter engages a blood vessel to that side of the catheter.

Both types of catheters must resist kinking in use. However kinking is exacerbated by the fact that because the catheters are made to be flexible at body temperature, the point of exit from the incision can be subject to maximum bending where the cool outer part meets the warmer and more flexible inserted part. The degree of kinking will of course also depend on the structure of the catheter. The double-D has some resistance in one plane as a result of the septum maintaining the distance between the septum connections to the outer wall, and the coaxial catheter can be improved by using more resistant tubing for both the inner and outer tubes. However there is clearly a need for a catheter which overcomes the inherent difficulties and disadvantages of both types.

In general, it is desirable in catheters of this type to provide a smooth exterior which contains two lumens with minimal flow resistance and structured to minimize the likelihood of kinking caused by bending forces, particularly at the site of the incision. The structure should also be designed to permit simple connection at the proximal end to tubing required to couple the catheter to apparatus such as a dialysis machine. At the distal end, the return lumen should be uninterrupted in cross-section if possible. A round cross-section is most desirable and if this were to continue throughout the catheter without interruption then the flow losses and turbulence would be minimized.

### Summary of the Invention

It has been found that structures according to the invention either meet the aforementioned requirements or at least minimize many of the problems associated with prior art catheters. Accordingly in one of its aspects, the invention provides a catheter of the type comprising a main body defining parallel side-by-side lumens and extending between a junction providing connecting tubes at the proximal end of the main body and a tip section forming an extension of the return lumen, and intake openings and return openings the catheter being characterized by the main body having a generally elliptical cross-section about major and minor axes and the intake and return lumens being centred on the major axis and separated by a wall, the wall extending along the minor axis and extending into reinforcement zones made up of solid catheter material and extending in the direction of the major axis and blending into lumen walls which extend across the major axis to enclose the lumens, each of the lumen walls being attached to both of the reinforcement zones.
FIG. 1 is a diagrammatic perspective view of a catheter according to a preferred embodiment of the invention and having a portion cut away to minimize the length of the catheter in the drawings;
FIG. 2 is a sectional view on line 2-2 of Fig. 1, and drawn to a larger scale than Fig. 1;
FIG. 3 is a figure similar to Fig. 2 taken on line 3-3 of FIG. 1; and
FIG. 4 is a longitudinal sectional view on line 4-4 of part of FIG. 1 and also drawn to a larger scale than that used for Fig. 1.

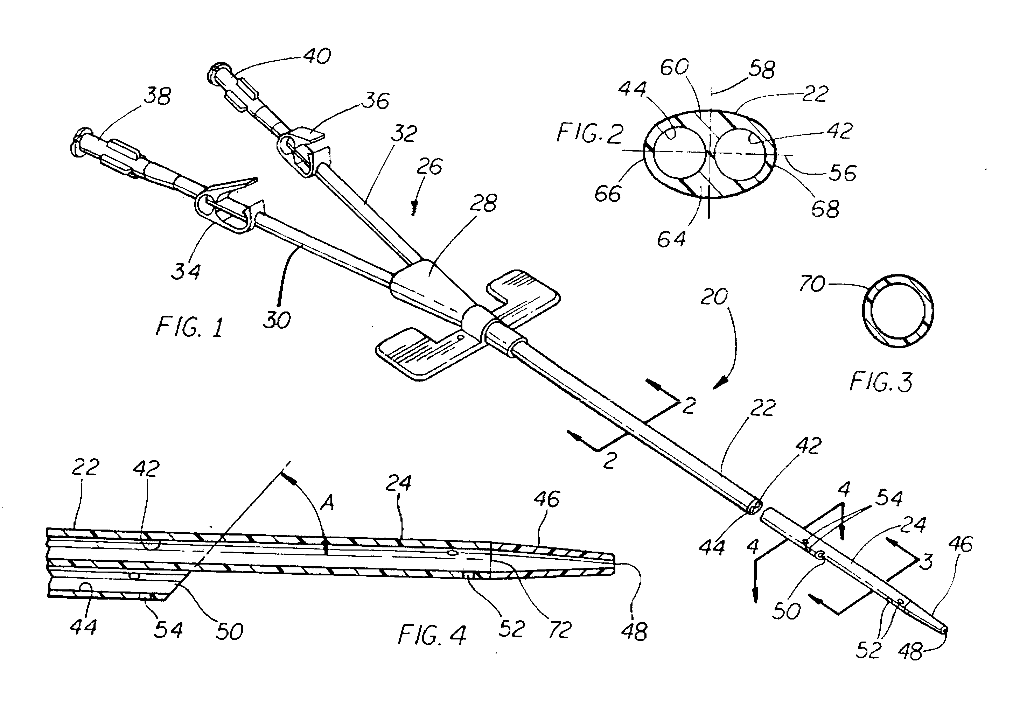

As seen in Fig. 1, a catheter 20 is provided which embodies the invention and is both a preferred embodiment of the invention and exemplary of other embodiments which could be made incorporating the inventive concept.

The catheter 20 includes a main body 22, tip section 24, and proximal end assembly 26 which has a junction 28 leading from the main body to individual connecting tubes 30, 32 having the usual clamps 34, 36 and terminating at respective luer connectors 38, 40. The tubes lead through the junction 23 to individual lumens 42, 44 in the main body. The return lumen 42 continues without interruption from the main body past the distal end of the main body and through the tip section 24 to a tapered tip 46 where it continues and converges to end at end opening 48. The intake lumen 44 terminates at a transverse opening 50 spaced from the end of the catheter as is usual practice to provide a separation between intake through the opening 50 and return through the end opening 48 in the same blood vessel.

The tip section 24 also includes side openings 52 and similarly, the intake lumen 44 has side openings 54 adjacent the opening 50 to enhance flow into the intake lumen 44.

As seen in FIG. 2, (which is drawn to enlarge the structure), the main body 22 has a generally elliptical outer shape or cross-section and contains the lumens 42, 44 which are round in cross-section and separated by a thin wall 55. The major and minor axes 56, 58 of the elliptical shape preferably meet at the centre of the wall 55 which extends along the minor axis opening out into reinforcement zones 60, 64 made up of solid catheter material. These portions then extend about adjacent parts of the lumens 42, 44 which are further defined by respective walls 66, 68 which cross the major axis 56 of the elliptical cross-section. The advantages and the utility of this cross-section will be explained in more detail once the description of the catheter has been completed,

As seen in FIG. 3, the cross-section of the tip section 24 is circular and annular. The lumen 42 is uninterrupted as it extends through the main body 22 continues through the tip section where it is surrounded by a circular wall 70 made as will be described. At the distal end, the tip section includes the tapered tip 46 which is preferably bonded in place as will be described to permit the use of a different hardness of material at the tip to enhance the flexibility of this part of the catheter if desired. Of course an integral tip can be used.

FIG. 4 is drawn to a larger scale than FIG. 1 and illustrates a longitudinal cross-section of the tip section and adjacent part of the main body 22. Several features can be seen in this view. First of all a separate tip 46 is seen to be tapered to the end opening 48 and as mentioned, is preferably of a different material from the rest of the tip section which is integral with the main body. The line 72 is intended to illustrate the joint between the materials. The structure starts as an extrusion in the form of Figure 2 which, after being cut to length, is modified by first cutting a portion of the lumen 44 to create the opening 50 and then slicing longitudinally to remove part of the lumen towards the distal end. Subsequently the projecting portion containing lumen 42 is placed in a mould where the thermoplastic material is deformed into the section shown in Fig. 3. Before this is done a mandrel is placed inside the lumen 42 to maintain the integrity and shape of this lumen and to ensure that it remains smooth walled throughout. The tip 46 is then bonded to the end of the remainder of the lumen 42 again using a mandrel to ensure proper alignment and shape.

The next step is to form the openings 52, 54 using conventional techniques. The proximal end assembly is then attached and this again can be done very simply due to the fact that the lumens 42, 44 are round and can accommodate attachment to the round tubing 30, 32 within the junction 28 as is now common in the art.

It will also be seen in FIG. 4 that the transverse opening 50 extends essentially transversely of the catheter but that the entrance is cut at an angle to the axis of the catheter. The enclosed "A" angle between the face of the opening and the axis of the catheter is preferably no smaller than 30°. This helps to ensure that the catheter will not be occluded by a blood vessel wall drawn by suction towards the opening. The blood vessel cannot accommodate the change in shape at this portion and will therefore be draped across the opening leaving access to the opening. If the angle becomes too small, then the blood vessel can change shape and occlude the opening as it is drawn into position by the suction applied by the dialysis apparatus attached to the catheter.

Returning to Fig. 2, the enhanced characteristics of the catheter are largely related to the arrangement of the cross-section shown in this figure. Firstly, the lumens 42, 44 have the best shape for flow, that is they are round in cross-section. It is well established in fluid mechanics that the surface area of contact is minimized relative to the volume of flow if a circular cross-section is used and laminar flow is more readily induced. Secondly, the rigidity of the structure to avoid kinking is significant. This can be demonstrated by an analogy with the bending of straight beams. If a bending force is applied to bend about the minor axis fig, the stress p is proportional to the distance along the major axis, y, and inversely proportional to the moment of inertia I about the minor axis 58. In other words, using standard beam formula for straight beams,

Pαy_{I}

From this it is evident that if the ratio y/I is the same about the x and y axes, the stress created at the surface will be the same for a given bending moment. It has been found that this desirable result can be approximated using the present structure. For example, consider a cross-section such as that shown in Fig. 2, having a major axis 56= 4.8 mm; the minor axis = 3.5 mm; the lumens are 1.78 mm in diameter; and the wall between the lumens is a minimum thickness of 0.5 mm. The ratio of the y/I for the x axis is 54864 cm cubed and y/I for the y axis is 51144 cm cubed. As a result this catheter (which is dimensioned for use in haemodialysis) will resist bending to approximately the same extent whether it is subjected to bending forces about the x or about the y axis.

As a result, the catheter provides a smooth outer surface containing lumens of round cross-section and having significant resistance to bending no matter which direction the bending force is applied. The overall cross-section is relatively small and the catheter is capable of being entered either using a sheath or, with a suitable mandrel in the intake lumen to occlude this lumen, the catheter can be entered over a Seldinger wire.

It will be evident that the dimension of the minor axis 58 can be increased to increase the resistance to bending about axis 56. However such a change will also increase the resistance to bending about the other axis. This means that for the two resistances to be equal the catheter will have to have a larger cross-section area. As a result it is preferable to optimize the design to maximize all advantages and it has been found that the example given above is acceptable. This can be stated in a relative way as follows.

The major axis of the cross-section is equal to the sum of the diameters of the lumens plus the thicknesses of the central dividing wall 55, and walls 66,68, and the minor axis is about 70 to 80 percent of the major axis. This ratio ensures that the reinforcement zones 60,64 are sufficient to achieve the desired resistance to bending.

Another relationship is that between the diameters of the lumens and the length of the major axis. The diameter of each lumen is in the order of 30 to 40 percent of the length of the major axis.

It will be appreciated that the cross-sectional shape of the catheter can be changed within the scope of the invention to achieve a generally constant resistance to bending and that the elliptical cross-section is a preferred embodiment.

## Claims

1. A dual lumen catheter (20) of the type comprising a main body (22) defining parallel side-by-side lumens (42,44) and extending between a junction (28) providing connecting tubes (30,32) at the proximal end of the main body and a tip section (24) forming an extension of the return lumen, and intake openings (54) and return openings (48,52, the catheter being characterized by the main body (22) having a generally elliptical cross-section about major and minor axes (56,58) and the intake and return lumens (42,44) being centred on the major axis and separated by a wall (55), the wall extending along the minor axis and extending into reinforcement zones (60,64) made up of solid catheter material and extending in the direction of the major axis and blending into lumen walls (66,68) which extend across the major axis to enclose the lumens, each of the lumen walls being attached to both of the reinforcement zones.

2. A dual lumen catheter as claimed in claim 1 in which the tip section (24) further includes a tip (46) at the distal end of the catheter of a physically different material from that of the remainder of the tip section.

3. A dual lumen catheter as claimed in claim 1 in the length of the minor axis is in the range of about 70 to 80 percent of that of the major axis.

4. A dual lumen catheter as claimed in claim 1 in which the lumens are round in cross-section.
